# EUROPEAN PATENT SPECIFICATION

(11) **EP 2 575 748 B1**
(45) Date of publication and mention of the grant of the patent: **11.04.2018**
(21) Application number: 11720124.4
(22) Date of filing: 19.05.2011
(51) Int. Cl.: A61K 8/36, A61K 8/35, A61K 8/81, A61K 8/86, A61Q 17/04, A61K 8/39, A61K 8/87

(54) **SUNSCREEN COMPOSITION**
SONNENSCHUTZZUSAMMENSETZUNG
COMPOSITION D'ÉCRAN SOLAIRE

(30) Priority: 20.07.2010 IN MU20622010; 25.05.2010 IN MU16102010
(43) Date of publication of application: 10.04.2013
(73) Proprietor: Unilever N.V., 3013 AL Rotterdam (NL); Unilever PLC, London, Greater London EC4Y 0DY (GB)
(72) Inventor: DUGGAL, Charu, Bangalore 560 066 (IN); GAURAV, Kumar, Whitefield Bangalore 560 066 (IN); RAUT, Janhavi, Sanjay, Bangalore 560 066 (IN); CHAVAN, Mohan, Vijaykumar, Bangalore 560 066 (IN); VAIDYA, Ashish, Anant, Bangalore 560 066 (IN); KINI, Mridula, Mumbai 400 099 (IN)
(74) Representative: Corsten, Michael Allan
(86) International application number: PCT/EP2011/058176
(87) International publication number: WO 2011/147738

(56) References cited:
- EP-A1- 1 618 870
- EP-A1- 2 105 124
- WO-A1-2008/022946
- JONES C: "Multifunctional Synthetic Rheology Modifiers for Personal Care Formulations: More Than Just Thickeners", ROHM AND HAAS PERSONAL CARE,, 1 May 2005 (2005-05-01), pages 1-23, XP007909943,

## Description

### Field of the invention

The invention relates to a high Sun Protection Factor (SPF) / UV-A Protection Factor (UVAPF) sunscreen composition.

### Background of the invention

Solar radiation includes about 5% ultraviolet (UV) radiation, wavelength of which is between 200 nm and 400 nm. It is further classified into three regions: from 320 to 400 nm (UV-A), 290 to 320 nm (UV-B) and from 200 to 290 nm (UV-C). A large part of UV-C radiation is absorbed by the ozone layer. Scientific studies have indicated that exposure to UV-A and UV-B radiation for short period causes reddening of the skin and localized irritation, whereas continued and prolonged exposure can lead to sunburn, melanoma and formation of wrinkles. It is also reported that UV radiation causes significant damage to hair. Therefore, it is desirable to protect the skin and other keratinous substrates of the human body from the harmful effects of both, UV-A and UV-B radiation.

Various cosmetic preparations have been reported for preventing and/or protecting the skin from harmful effects of ultraviolet radiation. Numerous organic sunscreen agents capable of absorbing UV-A rays are reported in the field of cosmetics amongst which a particularly useful sunscreen is of the dibenzoylmethane class. Many UV-B sunscreens are also known and approved for safe use in personal care compositions for protection from UV-B radiation. Many cosmetic manufacturers prefer to include both UV-A and UV-B sunscreens in photoprotective compositions so as to provide protection over the entire range of UV radiation. Sun protection Factor (SPF) and UV-A protection factor (UVAPF) are commonly measured attributes of photoprotective compositions which indicate the protection that the skin gets from exposure to both UV-B and UV-A radiation.

Thus, cosmetic manufacturers try to provide consumers with products having higher and higher SPF/ UVAPF. One of the ways of achieving this is to incorporate high levels of UV-A and UV-B sunscreens. One disadvantage of this approach is the high cost associated with incorporation of high levels of sunscreens which are expensive. Further, there are safety and regulatory limitations on the upper limit of incorporation of these sunscreens. Sensory properties are also reported to get affected on incorporation of high levels of sunscreens. Hence, there is a problem of achieving high SPF/ UVAPF while keeping the total amount of sunscreens in the compositions relatively low.

Various publications on more effective sunscreen compositions have been reported. One such patent publication is US 2005/0063925 which discloses an oil-in-water photoprotective emulsion containing Gemini surfactants and associative polymers. The Gemini surfactant is used as an emulsifier which has at least one dimeric surfactant comprising two surfactant units, which may be identical or different, each consisting of a hydrophilic head and a hydrophobic tail and connected to each other, via the hydrophilic heads, by means of a spacer group. Further, this composition comprises at least one photoprotective system capable of screening out UV rays, containing at least one mineral nanopigment based on metal oxide, and optionally at least one organic, preferably hydrosoluble or liposoluble UV-A and/or UV-B screening agent, and at least one associative polymer comprising at least one C₈-C₄₀ fatty chain.

JP 2008 162 930 (Shiseido) discloses a oil-in-water type emulsion sunscreening cosmetic comprising (a) an oil-soluble ultraviolet absorber (b) a water-soluble thickener (e.g. acrylic water-soluble polymer), (c) a water-soluble ultraviolet absorber and (d) one or more kinds of hydrophilic nonionic surfactant selected from the group consisting of a fatty acid-PEG-glyceryl-based surfactant, a hydrogenated castor oil-based surfactant and a PEG-PPG alkyl ether-based surfactant.

WO 2008/022946 (Unilever) discloses that cosmetic compositions comprising dibenzoylmethane or its derivative and p-methoxycinnamic acid or its derivative can be stabilized by incorporating a combination of fatty alcohol ethoxylates and polyalkyleneglycol.

EP1618870 discloses sunscreen compositions comprising dibenzoylmethane derivatives, acrylate/stearyl methacrylate copolymers and 1,5% by weight of stearic acid. These publications are targeted to ensuring stability of the formulation while having the desired photoprotection and is not reported to enhance SPF / UVAPF. Any attempt at increasing the SPF/ UVAPF has been with the use of high amounts of sunscreen compounds.

Therefore there exists a need for a personal care composition comprising sunscreen agents preferably in low concentrations that are able to provide much higher SPF / UVAPF values as compared to known sunscreen compositions comprising such low levels of sunscreen agents. It is desirable, if the enhanced SPF / UVAPF benefit could be achieved through synergistic interaction of commonly used ingredients, thereby giving the desired photoprotection benefits at substantially low costs.

The present inventors have been working on solving this problem and have surprisingly found that cosmetic compositions comprising dibenzoylmethane or its derivative and optionally an oil soluble UV-B sunscreen when incorporated in a sunscreen composition along with a non-ionic surfactant of a select class along with a polymer of a select class or a select fatty acid, provides the enhanced SPF / UVAPF benefits while maintaining the desired sensorial properties when applied on the substrate of interest.

It is therefore an object of the present invention to obviate at least some drawbacks of the prior art and provide high SPF/ UVAPF photo-protective sunscreen composition.

Another object of the present invention is to provide high SPF/ UVAPF sunscreen composition without compromising on the desired skin sensorial properties.

Yet another object of the present invention is to achieve the above objects using relatively low amounts of sunscreen agents thereby keeping costs low.

### Summary of the invention

According to the first aspect of the present invention there is provided a high SPF and/or UVAPF solid sunscreen composition comprising less than 8% total organic sunscreens, the composition comprising,
a) 0.1 to 5 % w/w dibenzoylmethane or its derivative;
b) 0.1 to 5 % w/w non-ionic surfactant selected from the class of ethoxylates of fatty alcohol/ fatty acid with saturated carbon chain and having HLB greater than 15.5 or from the class of ethoxylates of fatty alcohol / fatty acid with unsaturated carbon chain with HLB greater than 12;
c) 0.1 to 0.5 % w/w of a polymer selected from the class of acrylate / R methacrylate copolymer or crosspolymer, or an acrylate / R-alkyl acrylate crosspolymer; or from 0.2 - 0.5 % w/w of a polymer selected from the class of copolymers of acryloyldimethyltaurate with vinyl pyrrolidone, methacrylate or R-alkyl acrylate, and
d) a cosmetically acceptable base,
where R is a C10-30 alkyl group; and
- wherein said cosmetically acceptable base comprises higher than 5 to a maximum of 25% w/w fatty acid;
- wherein dibenzoylmethane or its derivative are selected from 4-tert-butyl- 4'-methoxydibenzoylmethane, 2-methyldibenzoylmethane, 4-methyl-dibenzoyl-ethane, 4-isopropyldibenzoyl-methane, 4-tert- butyldibenzoylmethane, 2,4-dimethyldibenzoylmethane, 2,5-dimethyldibenzoylmethane, 4,4'-diisopropyl-dibenzoylmethane, 2-methyl 5-isopropyl-4'-methoxydibenzoylmethane, 2-methyl-5-tert-butyl-4'- methoxy-dibenzoyl methane, 2,4-dimethyl-4'- methoxy dibenzoylmethane or 2,6-dimethyl-4-tert-butyl-4'-methoxy-dibenzoylmethane;
- wherein the composition has a SPF equal to or higher than 15 or a UVAPF value higher than or equal to 4.

The composition of the first aspect of the invention is preferably a cream.

According to the second aspect of the invention there is provided a high SPF and/or UVAPF non-solid sunscreen composition comprising less than 8% total organic sunscreens, the composition comprising,
a) 0.1 to 5 % w/w dibenzoylmethane or its derivative;
b) 0.1 to 5 % w/wt non-ionic surfactant selected from the class of ethoxylates of fatty alcohol/ fatty acid with saturated carbon chain and having HLB greater than 15.5 or from the class of ethoxylates of fatty alcohol / fatty acid with unsaturated carbon chain with HLB greater than 12;
c) higher than 5% to a maximum of 25 % w/w fatty acid;
d) 0.1 to 0.5 % w/w of a polymer selected from the class of acrylate / R methacrylate copolymer or crosspolymer, or an acrylate / R-alkyl acrylate crosspolymer; or from 0.2 - 0.5 % w/w of a polymer selected from the class of copolymers of acryloyldimethyltaurate with vinyl pyrrolidone, methacrylate or R-alkyl acrylate, and
e) a cosmetically acceptable base,
wherein dibenzoylmethane or its derivative are selected from 4-tert-butyl- 4'-methoxydibenzoylmethane, 2-methyldibenzoylmethane, 4-methyl-dibenzoyl-ethane, 4-isopropyldibenzoyl-methane, 4-tert- butyldibenzoylmethane, 2,4-dimethyldibenzoylmethane, 2,5-dimethyldibenzoylmethane, 4,4'-diisopropyl-dibenzoylmethane, 2-methyl 5-isopropyl-4'-methoxydibenzoylmethane, 2-methyl-5-tert-butyl-4'- methoxy-dibenzoyl methane, 2,4-dimethyl-4'- methoxy dibenzoylmethane or 2,6-dimethyl-4-tert-butyl-4'-methoxy-dibenzoylmethane;
- wherein the composition has a SPF equal to or higher than 15 or a UVAPF value higher than or equal to 4.

The composition of the second aspect of the invention is preferably a lotion.

### Detailed description of the invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Numerical ranges expressed in the format "from x to y" are understood to include x and y. When for a specific feature multiple preferred ranges are described in the format "from x to y", it is understood that all ranges combining the different endpoints are also contemplated.

By "A Sunscreen Composition" as used herein, is meant to include a composition for topical application to sun-exposed areas of the skin and/or hair of mammals, especially humans. Such a composition may be generally classified as leave-on or rinse off, and includes any product applied to a human body for also improving appearance, cleansing, odor control or general aesthetics. The composition of the present invention can be in the form of a liquid, lotion, cream, foam, scrub, gel, soap bar or toner, or applied with an implement or via a face mask, pad or patch. Non-limiting examples of such sunscreen compositions include leave-on skin lotions, creams, antiperspirants, deodorants, lipsticks, foundations, mascara, sunless tanners and sunscreen lotions and wash-off shampoos, conditioners, shower gels, toilet bars,. "Skin" as used herein is meant to include skin on the face and body (e.g., neck, chest, back, arms, underarms, hands, legs, buttocks and scalp) and especially to the sun exposed parts thereof. The composition of the invention is also of relevance to applications on any other keratinous substrates of the human body other than skin e.g. hair where products may be formulated with specific aim of providing photoprotection.

The first aspect of the invention provides for a solid sunscreen composition. By a solid sunscreen composition is meant a composition that has a critical shear stress (apparent yield stress) as defined by H A Barnes (Handbook of Elementary Rheology, University of Wales Aberystwyth, 71-73 (2000)) of higher than 20 Pa, preferably higher than 50 Pa at 25°C. It is preferred that the solid composition has an apparent yield stress of less than 10 000 Pa at 25°C. It is preferred that the viscosity of solid compositions of the invention at critical shear stress is in the range of 1 000 to 500 000 Pa.s, preferably 1 000 to 100 000 Pa.s at 25°C. A preferred solid composition is a cream. Creams are usually known as soft solids. Thus, by the use of the term solid composition, in the present invention, are included soft-solid compositions.

The second aspect of the invention provides for a non-solid sunscreen composition. By a non-solid sunscreen composition is meant a composition that has a critical shear stress (apparent yield stress) of less than 100 Pa, preferably les than 20 Pa to 25°C. The apparent yield stress is preferably at least 5 Pa at 25°C. It is preferred that the non-solid composition has a viscosity at critical shear stress of less 1000 Pa.s at 25°C. A preferred non-soild composition is a lotion.

Definitions of lotion and cream have been given by Brummer (Rheology Essentials of Cosmetic and Food Emulsions, Springer-Verlag Berline Heidelberg, 81-83 (2006)). Therein creams are defined as those compositions which do not flow out from a container at 25°C when it is turned upside down. Lotions are those compositions which flow out from the container at 25°C when turned upside down.

By 'A High SPF sunscreen composition' is meant a composition that has an SPF equal to or higher than 15, preferably more than 20, more preferably higher than 25. By 'A High UVAPF sunscreen composition' is meant a composition that has a UVAPF equal to or higher than 4, preferably higher than 8.

The SPF and UVAPF are measured using the standard protocols ISO/WD 4445 and ISO/CD 24443 respectively.

It is preferred that high SPF/UVAPF is achieved using total UV-B sunscreens in the range of 0.1 to 6 %, preferably from 0.5 to 6 % by weight of the composition. It is an advantage of the present invention that the high SPF/UVAPF values are achieved by using low amount of total organic sunscreens. By low amount of total organic sunscreens is meant that the total amount of organic sunscreens is less then 8 %, preferably less than 7 %, further more preferably less than 6 % by weight of the composition.

The composition of the invention comprises 0.1 to 5 % dibenzoylmethane or its derivative. Preferred dibenzoylmethane derivative are selected from 4-tert-butyl-4'-methoxydibenzoylmethane, 2-methyldibenzoylmethane, 4-methyl-dibenzoyl-ethane, 4-isopropyldibenzoyl-methane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxy-dibenzoyl methane, 2,4-dimethyl-4'- methoxy dibenzoylmethane or 2,6-dimethyl-4-tert-butyl-4'-methoxy-dibenzoylmethane. The most preferred dibenzoylmethane derivative is 4-tert.-butyl-4'-methoxydibenzoylmethane. Dibenzoylmethane or its derivative is preferably present in 0.2 to 5 %, more preferably 0.4 to 4 %, by weight of the composition.

The composition of the invention preferably comprises 0.1 to 7 %, preferably from 0.5 to 6 % an oil soluble UV-B organic sunscreen by weight of the composition. The oil soluble UV-B organic sunscreen is preferably selected from the class of cinnamic acid, salicylic acid, diphenyl acrylic acid or derivatives thereof. A few of the preferred oil soluble UV-B sunscreens which are commercially available and useful for inclusion in the composition of the invention are Octisalate™ (octyl salicylate), Homosalate™ (3,3,5-trimethyleyclohexyl 2-hydroxybenzoate), Neo Heliopan™ (a range of organic UV filters including ethylhexyl methoxycinnamate (Neo Heliopan AV) and ethylhexyl salicylate (Neo Heliopan OS)), Octocrylene™ (2-ethylhexyl 2-cyano-3,3-diphenyl-2-propenoate) or Parsol MCX™ (2-ethylhexyl-4-methoxycinnamate). According to a particularly preferred aspect of the invention the oil soluble UVB sunscreen is 2-ethylhexyl-4-methoxycinnamate. According to another particularly preferred aspect of the invention the oil soluble UVB sunscreen is 2-ethylhexyl 2-cyano-3,3-diphenyl-2-propenoate. It is interesting to note that only use of oil soluble UV-B sunscreens in the composition of the present invention provide the enhanced SPF/UVAPF benefits of the invention while water-soluble UV-B sunscreens do not provide the desired benefits. It is preferred that the composition of the invention is substantially free of water soluble organic sunscreens. Water soluble sunscreens may however be incorporated in small amount, preferably less than 2 %, further more preferably less than 1 %, and optimally absent from the composition of the invention.

An especially preferred oil soluble UVB sunscreen is 2-ethylhexyl 2-cyano-3,3-diphenyl-2-propenoate which is commercially available as Octocrylene™.

An important ingredient that contributes to enhancement of SPF/UVAPF of the sunscreen composition of the invention is a selective class of non-ionic surfactant. The non-ionic surfactant is selected from the class of ethoxylates of fatty alcohol/ fatty acids with saturated carbon chain and having HLB greater than 15.5 or from the class of ethoxylates of fatty alcohol/ fatty acids with unsaturated carbon chain with HLB greater than 12. It is observed that use of ionic surfactants or non-ionic surfactants of the fatty alcohol/ fatty acid ethoxylate class not meeting the claimed criteria do not provide the desired SPF/UVAPF enhancement. HLB is calculated using the Griffin method wherein HLB = 20 x Mh / M wherein Mh is the molecular mass of the hydrophilic portion of the molecule and M is the molecular mass of the whole molecule, giving a result on an arbitrary scale of 0 to 20. Typical values for various surfactants are given below:
- A value <10 : Lipid soluble (water insoluble)
- A value >10 : Water soluble
- A value from 4 to 8 indicates an anti-foaming atent
- A value from 7 to 11 indicates a W/O (water in oil) emulsifier
- A value from 12 to 16 indicates oil in water emulsion
- A value from 11 to 14 indicates a wetting agent
- A value from 12 to 15 is typical of detergents
- A value of 16 to 20 indicates a solubiliser or hydrotrope.

Ethoxylates of fatty alcohol (usually available under the Brij class) have the structure: 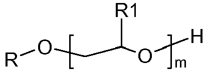 where R is a group consisting of carbon chain of 2 to 24; and R1 is alkyl or H.

Ethoxylates of the fatty acid (usually available under the Myrj class) have the structure: 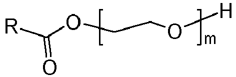 where R is a group consisting of carbon chain of 2 to 24.

Suitable examples of commercially available non-ionic surfactants for use in the composition of the invention are Brij 35 (also known as Laureth-23 or HO-(C₂H₄O)₂₃C₁₂H₂₅ or polyoxyethylene lauryl ether (a C12EO23 compound)), Brij 97 (also known as Oleth-10 or HO-(C₂H₄O)₁₀C₁₈H₃₅ or polyoxyethylene 10 oleyl ether (unsaturated C18EO10)), Brij 700 (also known as steareth-100 or polyoxyethylene (100) stearyl ether (C18EO100)), Brij 99 (polyoxyethylene (20) oleyl ether (unsaturated C18EO20)), Myrj S40 (PEG-40 stearate), Myrj S50 (PEG-50 stearate), or Myrj 59 (PEG-100 stearate). The non-ionic surfactant is included in 0.1 to 5 %, preferably 0.5 to 4 %, by weight of the composition.

Suitable polymers of the class of acrylate / R-methacrylate copolymer or crosspolymer, or an acrylate / R-alkyl acrylate crosspolymer, wherein R and alkyl are C₁₀₋₃₀ alkyl groups, include: (i) Acrylate/Beheneth-25 Methacrylate Copolymer, (ii) Acrylate/Steareth-20 Methacrylate Copolymer, (iii) Acrylate/Steareth-20 Methacrylate Crosspolymer, or (iv) Acrylates/C10-30 Alkyl Acrylate Crosspolymer.

R-methacrylate copolymer or crosspolymer has the general structure:

-(CH₂-CMeCO₂H)ₘ-(CH₂-CO₂R)ₙ-

R-alkyl acrylate crosspolymer has the general structure:

-(CH₂-CAlkylCO₂H)ₘ-(CH₂-CO₂R)ₙ-

wherein m and n are any whole integers in the range 500 - 10 000 000, preferably 500 - 1 000 000, most preferably 500 - 500 000.

Commercially available polymers useful for inclusion in the composition of the invention are Aculyn™ 22, Aculyn™ 28, or Aculyn™ 88, which are available from Rohm and Haas or Pemulen TR-2 which is available from Lubrizol.

Commercially available polymers of the class of copolymers of acryloyldimethyltaurate with vinyl pyrrolidone, methacrylate or R-alkyl acrylate include Aristoflex AVC™, Aristoflex HMB™, Aristoflex BLV™, Aristoflex AVS™ available from Clariant. When included, polymers from the above classes give the benefits of the invention at more than 0.2 % up to 0.5% by weight of the composition.

Not wishing to be bound by theory, it is observed that inclusion of the specific non-ionic surfactant meeting the above criterion in the solid compositions of the invention provides the desired SPF/UVAPF enhancement, but adversely affects the sensory properties of the solid product when applied on the skin. It is only with inclusion of the polymers meeting the above criterion that the sensory properties are attained while maintaining the high SPF/UVAPF values. When polymers, not meeting the above criterion, are used, either the desired sensory properties are not achieved or the SPF/UVAPF enhancement is not attained.

The composition of the invention comprises a cosmetically acceptable base. The cosmetically acceptable bases are such as to have a product in preferably a cream, lotion, gel or emulsion format. A preferred format for the solid form of the composition is a cream, further more preferably one which has a vanishing cream base. Vanishing cream base is one which comprises 3 to 25 % w/w fatty acid. Optionally, the composition may comprise 0.1 to 10 % soap. The fatty acid is preferably a C10 to C22 fatty acid, more preferably a C16 to C18 fatty acid. Most preferably the fatty acids are stearic acid or palmitic acid or a mixture thereof and the soap is preferably the potassium salt of the fatty acid mixture. The fatty acid is often hystric acid which is substantially (generally about 90 to 95 %) a mixture of 45 % stearic acid and 55 % palmitic acid. Thus, inclusion of hystric acid and its soap to prepare compositions of the invention is within the scope of the present invention. It is particularly preferred that the composition comprises higher than 5 %, preferably higher than 7 %, more preferably higher than 10 % fatty acid. It has been observed that use of such high levels of fatty acid also contributes to the high SPF/UVAPF. The cosmetically acceptable base is usually from 10 to 99.9 %, preferably from 50 to 99 % by weight of the composition. The cosmetically acceptable base preferably includes water. Water is preferably included in 35 to 90 %, more preferably 50 to 85 %, further more preferably 50 to 80 % by weight of the composition.

Other useful sun-protective agents e.g. inorganic sunblocks may be preferably used in the present invention. These include, for example, zinc oxide, iron oxide, silica, such as fumed silica, or titanium dioxide. The total amount of sun block that is preferably incorporated in the composition according to the invention is from 0.1 to 10 % by weight of the composition.

The composition of the invention may additionally comprise a skin lightening agent. The skin lightening agent is preferably chosen from a vitamin B3 compound or its derivative e.g. niacin, nicotinic acid, niacinamide or other well known skin lightening agents e.g. aloe extract, ammonium lactate, azelaic acid, kojic acid, citrate esters, ellagic acid, glycolic acid, green tea extract, hydroquinone, lemon extract, linoleic acid, magnesium ascorbyl phosphate, vitamins like vitamin B6, vitamin B12, vitamin C, vitamin A, a dicarboxylic acid, resorcinol derivatives, hydroxycarboxylic acid like lactic acid and their salts e.g. sodium lactate, and mixtures thereof. Vitamin B3 compound or its derivative e.g. niacin, nicotinic acid, niacinamide are the more preferred skin lightening agent as per the invention, most preferred being niacinamide. Niacinamide, when used, is preferably present in an amount in the range of 0.1 to 10%, more preferably 0.2 to 5% by weight of the composition.

The composition according to the invention may also comprise other diluents. The diluents act as a dispersant or carrier for other materials present in the composition, so as to facilitate their distribution when the composition is applied to the skin. Diluents other than water can include liquid or solid emollients, solvents, humectants, thickeners and powders.

The composition of the invention may comprise a conventional deodorant base as the cosmetically acceptable carrier. By a deodorant is meant a product in the stick, roll-on, or propellant medium which is used for personal deodorant benefit e.g. application in the under-arm or any other area which may or may not contain anti-perspirant actives.

Deodorant compositions can generally be in the form of firm solids, soft solids, gels, creams, and liquids and are dispensed using applicators appropriate to the physical characteristics of the composition.

The compositions of the present invention can comprise a wide range of other optional components. The CTFA Cosmetic Ingredient Handbook, Second Edition, 1992, which is incorporated by reference herein in its entirety, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents, and skin healing agents.

According to another aspect of the present invention there is provided use of a composition of the first aspect of the invention for obtaining SPF equal to or higher than 15 or UVAPF equal to or higher than 4. The SPF is preferably higher than 20, more preferably higher than 25. The UVAPF is preferably higher than 8. The use is preferably for non-therapeutic benefits.

The invention is now further described by way of the following non-limiting examples.

### Examples

### Examples 1 to 11: Effect of various non-ionic surfactants of the fatty alcohol ethoxylate class

Various compositions (in cream format) as shown in table 1 were prepared using different non-ionic surfactants. The difference between the various compositions were in the type of non-ionic surfactant used which are detailed in table 2. The SPF of the various compositions (examples 1-11) were measured and the results are shown in table 2. Invitro-SPF was measured using the Optometrics 290S instrument model. The substrate used was a 7.6 cm Transpore tape procured from 3M Company. The sample was applied at 2 mg/cm².

**Table 1**

| Ingredients | wt% |
|---|---|
| Hystric Acid | 17.00 |
| Parsol MCX™ | 2.25 |
| Parsol 1789™ | 1.20 |
| Non-ionic surfactant | 2.00 |
| Polymer^{#}, Aculyn 28™ | 0.40 |
| Niacinamide | 1.25 |
| Glycerine | 1.00 |
| Titanium dioxide | 0.90 |
| Potassium hydroxide | 0.57 |
| Silicone oil | 0.50 |
| Perfume | 0.33 |
| Methyl paraben + propyl paraben | 0.30 |
| Water | To 100 |

| | |
|---|---|
| # The polymer is available as a 20% dispersion in water and the dispersion is added at 2% into the composition. | |

**Table 2**

| Examples | Non-ionic surfactant | HLB of non-ionic surfactant | Carbon chain length of non-ionic surfactant | Number of EO groups on non-ionic surfactant | SPF |
|---|---|---|---|---|---|
| Example 1 | Brij -35 | 16.9 | 12 | 23 | 27.0 |
| Example 2 | Brij -700 | 18.8 | 18 | 100 | 29.0 |
| Example 3 | - | - | - | - | 9.7 |
| Example 4 | Brij -97 | 12.4 | 18 (unsaturated) | 10 | 26.0 |
| Example 5 | Brij-99 | 15.3 | 18 (unsaturated) | 20 | 30.0 |
| Example 6 | Brij -30 | 9.7 | 12 | 4 | 12.6 |
| Example 7 | Brij -56 | 12.9 | 16 | 10 | 13.5 |
| Example 8 | Brij-76 | 12.0 | 18 | 10 | 12.0 |
| Example 9 | Brij -52 | 5.0 | 16 | 2 | 10.0 |
| Example 10 | Brij -72 | 5.0 | 18 | 2 | 12.0 |
| Example 11 | Brij -92 | 4.9 | 18 (unsaturated) | 2 | 12.0 |

The sensory property in terms of skin feel on application to the skin was determined for example 1, 2 , 4 and 5 and this was compared to the skin feel of example 3. The study indicated that comparable skin feel is obtained for the examples 1, 2, 4 and 5 as compared to a sample outside the invention (example 3).

The data in table 2 indicates that compositions as per the invention (examples 1, 2, 4 and 5) which meet the selective criteria of the non-ionic surfactant provide for vastly improved SPF, with no compromise on the skin feel, as compared to a composition without a non-ionic surfactant and polymer (example 3) and non-ionic surfactant not meeting the selective criteria (examples 6 to 11).

### Examples 12 to 26: Effect of different polymers

Various compositions (in cream format) as shown in table 3 were prepared using different polymers. The difference between these compositions were in the type and/or concentration of polymers which are shown in table 4. These compositions were applied on the skin by a panel of experts and the summary of the sensory perception is given in table 4.

**Table 3**

| Ingredients | wt% |
|---|---|
| Hystearic Acid | 17.00 |
| Parsol MCX™ | 2.25 |
| Parsol 1789™ | 1.20 |
| Non-ionic surfactant Brij - 35 | As in table 4 |
| Polymer | As in table 4 |
| Niacinamide | 1.25 |
| Glycerine | 1.00 |
| Titanium dioxide | 0.90 |
| Potassium hydroxide | 0.57 |
| Silicone oil | 0.50 |
| Perfume | 0.33 |
| Methyl paraben + propyl paraben | 0.30 |
| Water | To 100 |

**Table 4**

| Examples | Wt% of Brij-35 | Polymer | Type of polymer | Wt% of polymer | Sensory perception |
|---|---|---|---|---|---|
| Example 1 | 2.0 | Aculyn - 28 | HASE | 0.25 | Acceptable - Comparable to example 3 |
| Example 3 | - | - | - | - | Commercially used with accepted sensory properties |
| Example 12 | 2.0 | - | - | - | Unacceptable - Very thin and soft |
| Example 13 | - | Aculyn - 28 | HASE | 0.30 | Unacceptable - harder than example 3 |
| Example 14 | 2.0 | Aculyn - 28 | HASE | 0.20 | Acceptable - Comparable to example 3 |
| Example 15 | 2.0 | Aculyn - 22 | HASE | 0.30 | Acceptable - Comparable to example 3 |
| Example 16 | 2.0 | Aculyn - 88 | HASE | 0.30 | Acceptable - Comparable to example 3 |
| Example 17 | 2.0 | Aculyn - 33 | ASE | 0.45 | Unacceptable - Softer than example 3 |
| Example 18 | 2.0 | Aculyn - 44 | HEUR | 0.50 | Unacceptable - Softer than example 3 |
| Example 19 | 2.0 | Aculyn - 46 | HEUR | 0.30 | Unacceptable - Softer than example 3 |
| Example 20 | 2.0 | Natrasol - 330 | HMHEC | 0.10 | Unacceptable - similar to example 10 |
| Example 21 | 2.0 | Aristoflex AVC | @ | 0.3 | Acceptable - Comparable to example 3 |
| Example 22 | 2.0 | Aristoflex HMB | @@ | 0.3 | Acceptable - Comparable to example 3 |
| Example 23 | 2.0 | Xanthan Gum | # | 1.0 | Unacceptable - Thicker and harder than example 3 |
| Example 24 | 2.0 | Microcrystalline cellulose | ## | 1.0 | Unacceptable - Softer than Example |
| Example 25 | 2.0 | U-15 | && | 1.0 | Unacceptable - Softer than Example |
| Example 26 | 2.0 | Pemulen TR-2 | ^^ | 0.2 | Acceptable - Comparable to Example 3. |

Some of the polymers, in table 4 are commercially available as a dispersion in water but the concentration as given in table 4 is on dry weight basis.

The types of polymers used were as follows:
HASE - Hydrophobically modified alkali soluble emulsion which is an acrylate / R-methacrylate copolymer or crosspolymer.
ASE - Alkali soluble emulsion
HEUR - Hydrophobically modified Ethylene Oxide Urethane
HMHEC - Hydrophobically modified Hydroxy ethyl cellulose
@ - Ammonium Acryloyldimethyltaurate/ VP copolymer
@@ - Ammonium Acryloyldimethyltaurate/ Beheneth - 25 Methacrylate crosspolymer
# - Naturally occurring polysaccharide
## - Made from naturally occurring cellulose
&& - U-15 which is a Poly Vinyl Pyrollidone Copolymer available from Induchem.
^^ - Pemulen TR -2 is an acrylate / C10-30 alkyl acrylate crosspolymer.

The data in table 4 indicates that use of a polymer as per the invention along with the selective non-ionic surfactant (examples 1, 14-16, 21,22 and 26) provides for the desired sensory properties while use of only the polymer alone, or only the surfactant alone or use of polymer outside the invention gives unacceptable sensory properties. (examples 12, 13, 17 to 20 and 23 to 25).

### Example 27 to 29: Effect of amount of fatty acid

Various compositions (in lotion format) were prepared as shown in table 5. They were then tested for SPF similar to the method used for measuring SPF of example 1. The data is summarized in table 5.

**Table 5**

| Ingredients | Example 27 wt% | Example 28 wt% | Example 29 wt% |
|---|---|---|---|
| Hystric Acid | 10.00 | 15.00 | 20.00 |
| Parsol MCX™ | 2.25 | 2.25 | 2.25 |
| Parsol 1789™ | 1.20 | 1.20 | 1.20 |
| Non-ionic surfactant Brij - 35 | 2.00 | 2.00 | 2.00 |
| Potassium Hydroxide | 0.60 | 0.60 | 0.60 |
| Water | To 100 | To 100 | To 100 |
| SPF | 15.0 | 24.0 | 28.0 |

The data in table 5 indicates that use of higher levels of hystric acid provides for the high desirable values of SPF.

### Examples 30 to 34: Effect of various non-ionic surfactants of the fatty acid ethoxylate class

Various compositions as shown in table 1 were prepared using different non-ionic surfactants. The difference between the various compositions were in the type of non-ionic surfactant used which are detailed in table 6. The SPF of the various compositions (examples 30-34) were measured and the results are shown in table 6. Invitro-SPF was measured using the Optometrics 290S instrument model. The substrate used was a 7.6 cm Transpore tape (3M Company). The sample was applied at 2 mg/cm².

**Table 6**

| Examples | Non-ionic surfactant | HLB of non-ionic surfactant | Surfactant common name | SPF |
|---|---|---|---|---|
| Example 3 | - | - | - | 9.7 |
| Example 30 | Myrj S8 (Myrj 52) | 10.8 | PEG-8 Stearate | 8.4 |
| Example 31 | Myrj S20 | 15.0 | PEG-20 Stearate | 12.0 |
| Example 32 | Myrj S40 | 16.7 | PEG-40 Stearate | 22.0 |
| Example 33 | Myrj S50 | 17.9 | PEG-50 Stearate | 29.0 |
| Example 34 | PEG-100 stearate | 18.8 | PEG-100 Stearate | 29.0 |

The data in table 6 indicates that compositions as per the invention (examples 32 to 34) which meet the selective criteria of the non-ionic surfactant provide for vastly improved SPF as compared to a composition without a non-ionic surfactant and polymer (example 3) and non-ionic surfactant not meeting the selective criteria (examples 30 and 31).

### Examples 35 to 36: Further cream compositions as per the invention for UVAPF benefit:

Compositions in cream format as shown in table 7 were prepared and the invitro UVAPF values were measured. The data is summarized in table 7.

**Table 7**

| Ingredients | Example 35 wt% | Example 36 wt% |
|---|---|---|
| Hystric Acid | 15.00 | 15.00 |
| Parsol 1789™ | 3.50 | 3.50 |
| Non-ionic surfactant Brij - 35 | - | 3.00 |
| Polymer, Aculyn 28 | 0.25 | 0.25 |
| Potassium hydroxide | 0.60 | 0.60 |
| Water | To 100 | To 100 |
| UVAPF | 3.7 | 8.2 |

The data provided in table 7 indicates that compositions as per the invention (example 36) provides for enhanced UVAPF values as compared to a composition outside the invention (example 35).

The invention thus provides for a high SPF/UVAPF sunscreen composition comprising relatively low amount of sunscreen compounds while having acceptable sensorial properties.

## Claims

1. A high SPF and/or UVAPF solid-sunscreen composition comprising less than 8% total organic sunscreens, the composition comprising:
a) 0.1 to 5 % w/w dibenzoylmethane or its derivative;
b) 0.1 to 5 % w/w non-ionic surfactant selected from the class of ethoxylates of fatty alcohol/ fatty acid with saturated carbon chain and having HLB greater than 15.5 or from the class of ethoxylates of fatty alcohol / fatty acid with unsaturated carbon chain with HLB greater than 12;
c) 0.1 to 0.5 % w/w of a polymer selected from the class of acrylate / R-methacrylate copolymer or crosspolymer, or an acrylate / R-alkyl acrylate crosspolymer; or from 0.2 - 0.5 % w/w of a polymer selected from the class of copolymers of acryloyldimethyltaurate with vinyl pyrrolidone, methacrylate or R-alkyl acrylate, and
d) a cosmetically acceptable base,
where R is a C₁₀₋₃₀ alkyl group; and
- wherein said cosmetically acceptable base comprises higher than 5 to a maximum of 25% w/w fatty acid;
- wherein dibenzoylmethane or its derivative are selected from 4-tert-butyl-4'-methoxydibenzoylmethane, 2-methyldibenzoylmethane, 4-methyl-dibenzoyl-ethane, 4-isopropyldibenzoyl-methane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxy-dibenzoyl methane, 2,4-dimethyl-4'- methoxy dibenzoylmethane or 2,6-dimethyl-4-tert-butyl-4'-methoxy-dibenzoylmethane;
- wherein the composition has a SPF equal to or higher than 15 or a UVAPF value higher than or equal to 4.

2. A composition as claimed in claim 1 wherein said cosmetically acceptable base is a cream.

3. A composition as claimed in any one of the preceding claims wherein said cosmetically acceptable base comprises 0.1 to 10 % w/w soap.

4. A high SPF and/or UVAPF non-solid sunscreen composition comprising less than 8% total organic sunscreens, the composition comprising,
a) 0.1 to 5 % w/w dibenzoylmethane or its derivative;
b) 0.1 to 5 % w/w non-ionic surfactant selected from the class of ethoxylates of fatty alcohol/ fatty acid with saturated carbon chain and having HLB greater than 15.5 or from the class of ethoxylates of fatty alcohol / fatty acid with unsaturated carbon chain with HLB greater than 12;
c) higher than 5 to a maximum of 25 % w/w fatty acid;
d) 0.1 to 0.5 % w/w of a polymer selected from the class of acrylate / R-methacrylate copolymer or crosspolymer, or an acrylate / R-alkyl acrylate crosspolymer; or from 0.2 - 0.5 % w/w of a polymer selected from the class of copolymers of acryloyldimethyltaurate with vinyl pyrrolidone, methacrylate or R-alkyl acrylate, where R is a C10-30 alkyl group; and
e) a cosmetically acceptable base;
wherein dibenzoylmethane or its derivative are selected from 4-tert-butyl-4'-methoxydibenzoylmethane, 2-methyldibenzoylmethane, 4-methyl-dibenzoyl-ethane, 4-isopropyldibenzoyl-methane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxy-dibenzoyl methane, 2,4-dimethyl-4'- methoxy dibenzoylmethane or 2,6-dimethyl-4-tert-butyl-4'-methoxydibenzoylmethane; and
wherein the composition has a SPF equal to or higher than 15 or a UVAPF value higher than or equal to 4.

5. A composition as claimed in claim 4 wherein said cosmetically acceptable base is a lotion.

6. A composition as claimed in any one of the preceding claims wherein said non-ionic surfactant is selected from the group consisting of Brij 35 (Laureth-23 or HO-C₂H₄O)₂₃C₁₂H₂₅ or polyoxyethylene lauryl ether), Brij 97 (Oleth-10 or HO-(C₂H₄O)₁₀C₁₈H₃₅ or polyoxyethylene 10 oleyl ether), Brij 700 (steareth-100 or polyoxyethylene (100) stearyl ether), Brij 99 (polyoxyethylene (20) oleyl ether), Myrj S40 (PEG-40 stearate), Myrj S50 (PEG-50 stearate) and Myrj 59 (PEG-50 stearate).

7. A composition as claimed in any one of the preceding claims further comprising 0.1 to 7 % w/w an oil soluble UV-B sunscreen.

8. A composition as claimed in claim 7 wherein said oil soluble UV-B organic sunscreen is selected from the group consisting of cinnamic acid, salicylic acid, diphenyl acrylic acid and derivatives thereof.

9. A composition as claimed in claim 8 wherein the oil soluble organic sunscreen is selected from Octisalate™ (octyl salicylate), Homosalate™ (3,3,5-trimethyleyclohexyl 2-hydroxybenzoate), NeoHelipan™ (a range of organic UV filters including ethylhexyl methoxycinnamate and ethylhexyl salicylate), Octocrylene™ (2-ethylhexyl 2-cyano-3,3-diphenyl-2-propenoate) or Parsol MCX™ (2-ethylhexyl-4-methoxycinnamate).

10. A composition as claimed in any one of the preceding claims wherein said dibenzoyl methane derivative is 4-tert.-butyl-4'-methoxydibenzoylmethane.

## Patentansprüche

1. Feste Sonnenschutzzusammensetzung mit hohem SPF und/oder UVAPF, umfassend weniger als 8% gesamte organische Sonnenschutzmittel, wobei die Zusammensetzung umfasst:
a) 0,1 bis 5% Gew./Gew. Dibenzoylmethan oder seines Derivats,
b) 0,1 bis 5% Gew./Gew. nicht-ionisches Tensid, ausgewählt aus der Klasse von Ethoxylaten von Fettalkohol/Fettsäure mit gesättigter Kohlenstoffkette und mit einem HLB von größer als 15,5 oder aus der Klasse von Ethoxylaten von Fettalkohol/Fettsäure mit ungesättigter Kohlenstoffkette mit einem HLB von größer als 12,
c) 0,1 bis 0,5% Gew./Gew. eines Polymers, ausgewählt aus der Klasse von Acrylat/R-Methacrylat-Copolymer oder -Crosspolymer oder einem Acrylat/R-Alkylacrylat-Crosspolymer, oder von 0,2 bis 0,5% Gew./Gew. eines Polymers, ausgewählt aus der Klasse von Copolymeren von Acryloyldimethyltaurat mit Vinylpyrrolidon, Methacrylat oder R-Alkylacrylat, und
d) einen kosmetisch annehmbaren Grundbestandteil,
wobei R eine C₁₀₋₃₀-Alkylgruppe darstellt und
- worin der kosmetisch annehmbare Grundbestandteil mehr als 5 bis maximal 25% Gew./Gew. Fettsäure umfasst,
- wobei Dibenzoylmethan oder sein Derivat ausgewählt sind aus 4-tert-Butyl-4'-methoxydibenzoylmethan, 2-Methyldibenzoylmethan, 4-Methyldibenzoylethan, 4-Isopropyldibenzoylmethan, 4-tert-Butyldibenzoylmethan, 2,4-Dimethyldibenzoylmethan, 2,5-dimethyldibenzoylmethan, 4,4'-Diisopropyldibenzoylmethan, 2-Methyl-5-isopropyl-4'-methoxydibenzoylmethan, 2-Methyl-5-tert-butyl-4'-methoxydibenzoylmethan, 2,4-Dimethyl-4'-methoxydibenzoylmethan oder 2,6-Dimethyl-4-tert-butyl-4'-methoxy-dibenzoylmethan,
- wobei die Zusammensetzung einen SPF-Wert von gleich oder höher als 15 oder einen UVAPF-Wert von höher als oder gleich 4 aufweist.

2. Zusammensetzung, wie im Anspruch 1 beansprucht, wobei der kosmetisch annehmbare Grundbestandteil eine Creme ist.

3. Zusammensetzung, wie in irgendeinem der vorhergehenden Ansprüche beansprucht, wobei der kosmetisch annehmbare Grundbestandteil 0,1 bis 10% Gew./Gew. Seife umfasst.

4. Nicht-feste Sonnenschutzzusammensetzung mit hohem SPF und/oder UVAPF, umfassend weniger als 8% gesamte organische Sonnenschutzmittel, wobei die Zusammensetzung umfasst:
a) 0,1 bis 5% Gew./Gew. Dibenzoylmethan oder seines Derivats,
b) 0,1 bis 5% Gew./Gew. nicht-ionisches Tensid, ausgewählt aus der Klasse von Ethoxylaten von Fettalkohol/Fettsäure mit gesättigter Kohlenstoffkette und mit einem HLB von größer als 15,5 oder aus der Klasse von Ethoxylaten von Fettalkohol/Fettsäure mit ungesättigter Kohlenstoffkette mit einem HLB von größer als 12,
c) mehr als 5 bis maximal 25% Gew./Gew. Fettsäure,
d) 0,1 bis 0,5% Gew./Gew. eines Polymers, ausgewählt aus der Klasse von Acrylat/R-Methacrylat-Copolymer oder -Crosspolymer oder einem Acrylat/R-Alkylacrylat-Crosspolymer, oder von 0,2 bis 0,5% Gew./Gew. eines Polymers, ausgewählt aus der Klasse von Copolymeren von Acryloyldimethyltaurat mit Vinylpyrrolidon, Methacrylat oder R-Alkylacrylat, wobei R eine C₁₀₋₃₀-Alkylgruppe darstellt, und
e) einen kosmetisch annehmbaren Grundbestandteil,
wobei Dibenzoylmethan oder sein Derivat ausgewählt sind aus 4-tert-Butyl-4'-methoxydibenzoylmethan, 2-Methyldibenzoylmethan, 4-Methyldibenzoylethan, 4-Isopropyldibenzoylmethan, 4-tert-Butyldibenzoylmethan, 2,4-Dimethyldibenzoylmethan, 2,5-Dimethyldibenzoylmethan, 4,4'-Diisopropyldibenzoylmethan, 2-Methyl-5-isopropyl-4'-methoxydibenzoylmethan, 2-Methyl-5-tert-butyl-4'-methoxydibenzoylmethan, 2,4-Dimethyl-4'-methoxydibenzoylmethan oder 2,6-Dimethyl-4-tert-butyl-4'-methoxydibenzoylmethan,
wobei die Zusammensetzung einen SPF-Wert von gleich oder höher als 15 oder einen UVAPF-Wert von höher als oder gleich 4 aufweist.

5. Zusammensetzung, wie im Anspruch 4 beansprucht, wobei der kosmetisch annehmbare Grundbestandteil eine Lotion ist.

6. Zusammensetzung, wie in irgendeinem der vorhergehenden Ansprüche beansprucht, wobei das nicht-ionische Tensid aus der Gruppe ausgewählt ist, die aus Brij 35 (Laureth-23 oder HO-C₂H₄O)₂₃C₁₂H₂₅ oder Polyoxyethylenlaurylether), Brij 97 (Oleth-10 oder HO-(C₂H₄O)₁₀C₁₈H₃₅ oder Polyoxyethylen-10-oleylether), Brij 700 (Steareth-100 oder Polyoxyethylen(100)stearylether), Brij 99 (Polyoxyethylen(20)oleylether), Myrj S40 (PEG-40-stearat), Myrj S50 (PEG-50-stearat) und Myrj 59 (PEG-50-stearat) besteht.

7. Zusammensetzung, wie in irgendeinem der vorhergehenden Ansprüche beansprucht, ferner umfassend 0,1 bis 7% Gew./Gew. an öllöslichem UV-B-Sonnenschutzmittel.

8. Zusammensetzung, wie im Anspruch 7 beansprucht, wobei das öllösliche organische UV-B-Sonnenschutzmittel aus der Gruppe ausgewählt ist, die aus Zimtsäure, Salicylsäure, Diphenylacrylsäure und Derivaten davon besteht.

9. Zusammensetzung, wie im Anspruch 8 beansprucht, wobei das öllösliche organische Sonnenschutzmittel ausgewählt ist aus Octisalate^{Wz} (Octylsalicylat), Homosalate^{Wz} (3,3,5-Trimethylcyclohexyl-2-hydroxybenzoat), NeoHelipan^{Wz} (ein Bereich von organischen UV-Filtern, einschließlich Ethylhexylmethoxycinnamat und Ethylhexylsalicylat), Octocrylene^{Wz} (2-Ethylhexyl-2-cyano-3,3-diphenyl-2-propenoat) oder Parsol MCX^{Wz} (2-Ethylhexyl-4-methoxycinnamat).

10. Zusammensetzung, wie in irgendeinem der vorhergehenden Ansprüche beansprucht, wobei das Dibenzoylmethan-Derivat 4-tert-Butyl-4'-methoxydibenzoylmethan ist.

## Revendications

1. Composition d'écran solaire solide à SPF et/ou UVAPF élevé comprenant moins de 8 % d'écrans solaires organiques totaux, la composition comprenant :
a) de 0,1 à 5 % en masse/masse de dibenzoylméthane ou de son dérivé ;
b) de 0,1 à 5 % en masse/masse de tensioactif non ionique choisi dans la classe d'éthoxylates d'alcools gras/acides gras avec une chaîne carbonée saturé et ayant un HLB supérieur à 15,5 ou dans la classe d'éthoxylates d'alcools gras/acides gras avec une chaîne carbonée insaturée avec un HLB supérieur à 12 ;
c) de 0,1 à 0,5 % en masse/masse d'un polymère choisi dans la classe de copolymère ou polymère croisé d'acrylate/R-méthacrylate, ou d'un polymère croisé d'acrylate/acrylate de R-alkyle ; ou de 0,2-0,5 % en masse/masse d'un polymère choisi dans la classe de copolymères de taurate d'acryloyldiméthyle avec de la vinylpyrrolidone, de méthacrylate ou d'acrylate de R-alkyle, et
d) une base cosmétiquement acceptable,
où R est un groupe alkyle en C₁₀₋₃₀ ; et
- dans laquelle ladite base cosmétiquement acceptable comprend plus de 5 à un maximum de 25 % en masse/masse d'acide gras ;
- dans laquelle le dibenzoylméthane ou son dérivé sont choisis parmi le 4-tert-butyl-4'-méthoxydibenzoylméthane, 2-méthyldibenzoyl-méthane, 4-méthyldibenzoyl-éthane, 4-isopropyldibenzoyl-méthane, 4-tert-butyldibenzoylméthane, 2,4-diméthyldibenzoylméthane, 2,5-diméthyldibenzoylméthane, 4,4'-diisopropyl-dibenzoylméthane, 2-méthyl-5-isopropyl-4'-méthoxydibenzoylméthane, 2-méthyl-5-tert-butyl-4'-méthoxy-dibenzoylméthane, 2,4-diméthyl-4'-méthoxydibenzoylméthane ou 2,6-diméthyl-4-tert-butyl-4'-méthoxydibenzoylméthane ;
- dans laquelle la composition présente un SPF supérieur ou égal à 15 ou une valeur UVAPF supérieure ou égale à 4.

2. Composition selon la revendication 1, dans laquelle ladite base cosmétiquement acceptable est une crème.

3. Composition selon l'une quelconque des revendications précédentes, dans laquelle ladite base cosmétiquement acceptable comprend de 0,1 à 10 % en masse/masse de savon.

4. Composition d'écran solaire non-solide à SPF et/ou UVAPF élevé comprenant moins de 8 % d'écrans solidaires organiques totaux, la composition comprenant :
a) de 0,1 à 5 % en masse/masse de dibenzoylméthane ou de son dérivé ;
b) de 0,1 à 5 % en masse/masse de tensioactif non-ionique choisi dans la classe d'éthoxylates d'alcools gras/acides gras avec une chaîne carbonée saturée et ayant un HLB supérieur à 15,5 ou dans la classe d'éthoxylates d'alcools gras/acides gras avec une chaîne carbonée insaturée avec un HLB supérieur à 12 ;
c) plus de 5 à un maximum de 25 % en masse/masse d'acide gras ;
d) de 0,1 à 0,5 % en masse/masse d'un polymère choisi dans la classe d'un copolymère ou polymère croisé d'acrylate/R-méthacrylate, ou d'un polymère croisé d'acrylate/acrylate de R-alkyle ; ou de 0,2-0,5 % en masse/masse d'un polymère choisi dans la classe de copolymères de taurate d'acryloyldiméthyle avec de la vinylpyrrolidone, de méthacrylate ou d'acrylate de R-alkyle, où R est un groupe alkyle en C10-30 ; et
e) une base cosmétiquement acceptable ;
dans laquelle le dibenzoylméthane ou son dérivé sont choisis parmi le 4-tert-butyl-4'-méthoxydibenzoylméthane, 2-méthyldibenzoyl-méthane, 4-méthyldibenzoyl-éthane, 4-isopropyldibenzoyl-méthane, 4-tert-butyl-dibenzoylméthane, 2,4-diméthyldibenzoylméthane, 2,5-diméthyldibenzoyl-méthane, 4,4'-diisopropyl-dibenzoylméthane, 2-méthyl-5-isopropyl-4'-méthoxydibenzoylméthane, 2-méthyl-5-tert-butyl-4'-méthoxy-dibenzoylméthane, 2,4-diméthyl-4'-méthoxydibenzoylméthane ou 2,6-diméthyl-4-tert-butyl-4'-méthoxydibenzoylméthane ; et
dans laquelle la composition présente un SPF supérieur ou égal à 15 ou une valeur UVAPF supérieure ou égale à 4.

5. Composition selon la revendication 4, dans laquelle ladite base cosmétiquement acceptable est une lotion.

6. Composition selon l'une quelconque des revendications précédentes, dans laquelle ledit tensioactif non ionique est choisi dans le groupe constitué de Brij 35 (Laureth-23 ou HO-(C₂H₄O)₂₃C₁₂H₂₅ ou lauryléther de polyoxyéthylène), Brij 97 (Oleth-10 ou HO-(C₂H₄O)₁₀C₁₈H₃₅ ou oléyléther de polyoxyéthylène 10), Brij 700 (steareth-100 ou stéaryléther de polyéthylène (100)), Brij 99 (oléyléther de polyoxyéthylène (20)), Myrj S40 (stéarate de PEG-40), Myrj S50 (stéarate de PEG-50), et Myrj 59 (stéarate de PEG-50).

7. Composition selon l'une quelconque des revendications précédentes comprenant de plus de 0,1 à 7 % en masse/masse d'un écran solaire aux UV-B soluble dans l'huile.

8. Composition selon la revendication 7, dans laquelle ledit écran solaire organique aux UV-B soluble dans l'huile est choisi dans le groupe constitué d'acide cinnamique, d'acide salicylique, d'acide diphénylacrylique et de dérivés de ceux-ci.

9. Composition selon la revendication 8, dans laquelle l'écran solaire organique soluble dans l'huile est choisi parmi Octisalate™ (salicylate d'octyle), Homosalate™ (2-hydroxybenzoate de 3,3,5-triméthyleyclohexyle), NeoHelipan™ (une gamme de filtres UV organiques incluant le méthoxycinnamate d'éthylhexyle et le salicylate d'éthylhexyle), Octocrylene™ (2-cyano-3,3-diphényl-2-propénoate de 2-éthylhexyle) ou Parsol MCX™ (2-éthylhexyl-4-méthoxycinnamate).

10. Composition selon l'une quelconque des revendications précédentes, dans laquelle ledit dérivé de dibenzoylméthane est le 4-tert.-butyl-4'-méthoxydibenzoylméthane.
